Europäisches Patentamt

European Patent Office

Office européen des brevets

(11) Veröffentlichungsnummer: **0 357 585 B1**

(12) **EUROPÄISCHE PATENTSCHRIFT**

(45) Veröffentlichungstag der Patentschrift: **06.04.94**

(21) Anmeldenummer: **89890169.9**

(22) Anmeldetag: **19.06.89**

(51) Int. Cl.⁵: **A61K 39/104, A61K 39/40**

(54) Gegen Pseudomonas aeruginosa-Infektionen wirksame Präparationen sowie Immunglobulin-G-hältige, gegen Bakterium Pseudomonas aeruginosa wirksame Präparationen.

(30) Priorität: **29.08.88 AT 2113/88**

(43) Veröffentlichungstag der Anmeldung:
**07.03.90 Patentblatt 90/10**

(45) Bekanntmachung des Hinweises auf die
Patenterteilung:
**06.04.94 Patentblatt 94/14**

(84) Benannte Vertragsstaaten:
**AT BE CH DE ES FR GB IT LI LU NL SE**

(56) Entgegenhaltungen:
**EP-A- 0 252 064**
**WO-A-86/03974**

(73) Patentinhaber: **IMMUNO Aktiengesellschaft**
**Industriestrasse 67**
**A-1221 Wien(AT)**

(72) Erfinder: **Rotering, Heinz, Dr.**
**Lindenweg 22**
**D-6074 Rödermark-Waldacker(DE)**
Erfinder: **Eibl, Johann, Dr.**
**Gustav Tschermakgasse 2**
**A-1180 Wien(AT)**
Erfinder: **Dorner, Friedrich, Prof. Dr.**
**Peterlinigasse 17**
**A-1230 Wien(AT)**

(74) Vertreter: **Wolfram, Gustav, Dipl.-Ing.**
**Patentanwälte**
**Sonn, Pawloy, Weinzinger & Wolfram,**
**Riemergasse 14**
**A-1010 Wien (AT)**

**Beschreibung**

Die Erfindung betrifft gegen Pseudomonas aeruginosa-Infektionen wirksame Präparationen, insbesondere Vakzine, deren Wirksamkeit sich gegen alle Kombinationen der Partial-Flagella(H)-Antigene $a_0$, $a_1$, $a_2$, $a_3$, $a_4$ und b erstreckt und die zur aktiven Immunisierung bestimmt sind, sowie Immunglobulin-G-hältige, gegen Bakterium Pseudomonas aeruginosa wirksame Präparationen, die zum passiven Schutz bestimmt sind.

Das Bakterium Pseudomonas aeruginosa ist ein opportunistisch pathogener Keim, der häufig bei Krankenhausinfektionen auftritt, hauptsächlich bei Patienten mit geschwächter Immunabwehr, wie bei Verbrennungspatienten, bei Personen, die an cystischer Fibrose leiden oder organische Fehlfunktionen aufweisen, und bei Tumorpatienten. Antibiotika sind gegen Pseudomonasinfektionen infolge des Auftretens von Resistenzen nur beschränkt wirksam, weshalb man bemüht ist, immunologische Methoden zur Bekämpfung von Infektionen durch Pseudomonas aeruginosa zu finden.

Infektionen können durch eine Vielzahl von Stämmen ausgelöst werden, die durch O-Antigene (Lipopolysaccharid) und H-Antigene (Flagellen) charakterisiert werden können, wobei H- und O-Antigentypen frei kombinierbar sind.

Es ist bekannt, daß die Motilität von Pseudomonas aeruginosa zur Virulenz des Keimes beiträgt (Montie et al., Infect. Immunity 38, 1296-1298, 1982). Motile Pseudomonas aeruginosa-Stämme besitzen eine einzige Flagelle. Burned Mouse-Modelluntersuchungen haben ergeben, daß ein größerer Prozentsatz von Mäusen überlebt, wenn nicht-motile Pseudomonas aeruginosa-Stämme in experimentell erzeugte Verbrennungen inokuliert werden, als wenn motile Stämme verwendet werden (Montie et al., siehe oben). Weitere Untersuchungen über die Pathogenese von Pseudomonas aeruginosa haben erbracht, daß Tiere, die mit Flagellen-Antigenpräparationen immunisiert wurden, geschützt waren, wenn eine Verbrennung gesetzt und mit motilen Stämmen der Bakterien infiziert wurde (Holder et al., Infect. Immunity 35, 276-280, 1982).

Pseudomonas aeruginosa-Flagellen wurden mittels serologischer Methoden untersucht, und es wurde gezeigt, daß zwei Hauptantigentypen unterschieden werden können, die nach R. Ansorg (Zbl. Bakt. Hyg. I. Abt. Orig. A, 242, 228-238, 1978) als Typ a und Typ b bezeichnet werden.

Im Flagella(H)-Antigen vom Typ a können unter Verwendung der indirekten Immunfluoreszenztechnik die Partialantigene $a_0$, $a_1$, $a_2$, $a_3$ und $a_4$ differenziert werden, die von den Pseudomonas aeruginosa-Stämmen in unterschiedlichen Kombinationen exprimiert werden, wobei das Partialantigen $a_0$ auf allen Flagellen vom Typ a gefunden wird. Das Flagella(H)-Antigen vom Typ b verhält sich serologisch einheitlich, d.h. es können keine Untergruppen identifiziert werden.

Insgesamt lassen sich nach Ansorg bei Pseudomonas aeruginosa 17 H-Typen unterscheiden, u.zw. ein H-Typ b und 16 H-Typen des Flagella-Antigens a, bestehend aus dem in allen a-Typen vorhandenen Partialantigen $a_0$ und gegebenenfalls den Partialantigenen $a_1$ und/oder $a_2$ und/oder $a_3$ und/oder $a_4$. Eingehende Untersuchungen von Flagellen der Pseudomonas aeruginosa-Stämme 5142 und 13030, die von Ansorg als ausschließlich der Untergruppe $a_0$ zugehörig charakterisiert wurden, wiesen diese als biochemisch und immunologisch identisch mit Flagellen vom Typ b aus (Montie et al., Infect. Immunity 49(3), 770-774 (1985)).

Die Gewinnung von polydispersen nativen Flagella(H)-Antigenen (FAg) ist in der PCT-Veröffentlichung WO 86/03974 beschrieben. Dazu werden Bakterienkulturen in Minimalmedium angezüchtet und anschließend mit einem Detergens behandelt, wonach die FAg aus der Kultur abgetrennt werden. Dabei wird die Bakterienkultur vor der Detergensbehandlung desintegriert und einem Scherprozeß unterworfen. Durch den Zusatz von Detergens wird die Trennbarkeit des Antigens von der Bakterienmasse bewirkt; die sich dann in Suspension befindlichen Antigene können durch differentielle Zentrifugation von den Zellen abgetrennt werden.

Anschließend können die wie oben beschrieben von der Bakterienmasse abgetrennten Antigene durch eine chromatographische Reinigung von anhaftenden Verunreinigungen, wie Lipopolysacchariden, Nucleinsäuren, Salzen, Polysacchariden u.ä. befreit werden. Das noch vorhandene Detergens kann durch einen weiteren säulenchromatographischen Reinigungsschritt entfernt werden.

Die zur Bereitung von Pseudomonas aeruginosa-Bakterienkulturen verwendbaren Stämme und die produzierten Antigene sind in der folgenden Tabelle angeführt.

| Stamm | H-Typ | Hinterlegungsstelle und -nummer: |
|-------|-------|----------------------------------|
| M-2 | b | ATCC 33349 |
| 5142 | $a_0$ (b) | PCC 5142 |
| 5940 | $a_0 a_2$ | PCC 5940 |
| WR-5 | $a_0 a_2$ | *) |
| *) erhältlich bei: Dr.Barbara H. Iglewski, Department of Microbiology & Immunology, Oregon Health Sciences University, Portland, Oregon 97201, USA | | |

| Stamm | H-Typ | Hinterlegungsstelle und -nummer: |
|-------|-------|----------------------------------|
| 5939 | $a_0 a_3$ | PCC 5939 |
| 1210 | $a_0 a_1 a_2$ | ATCC 33354 |
| 170018 | $a_0 a_3 a_4$ | ATCC 33356 |

Der EP-A - 0 252 064 ist zu entnehmen, daß alle einzelnen Flagellenantigene der in der PCT-Veröffentlichung WO 86/03974 beschriebenen Typen als Bestandteile einer Vakzine protektive Wirkung besitzen und daß sich das Serum, das von Mäusen gewonnen wurde, die mit den in der PCT-Veröffentlichung WO 86/03974 angeführten Flagellenantigenen aktiv immunisiert worden waren, zur passiven Immunisierung von Mäusen eignet.

Allerdings konnte die protektive Wirkung der einzelnen Antigene und der von diesen abgeleiteten Antikörper nur im Tiermodell und nur im homologen System nachgewiesen werden, d.h. es konnte nur eine Wirkung gegen Keime desjenigen Flagellenserotyps gefunden werden, der zur Immunisierung Verwendung fand.

Ärztlicherseits besteht der Wunsch nach einer Pseudomonas aeruginosa-Vakzine, deren Wirksamkeit sich gegen alle Kombinationen der Partial-Flagella(H)-Antigene $a_0$, $a_1$, $a_2$, $a_3$, $a_4$ und b erstreckt, da eine Analyse von 300 klinischen Pseudomonas aeruginosa-Isolaten mit dem von Ansorg (Zbl. Bakt. Hyg. I. Abt. Orig. A242, 228-238, 1978) vorgeschlagenen H-Antigentypisierungsschema ergab, daß bei 98 % aller motilen Pseudomonas-Keime Partialantigene vom Typ a und/oder b nachgewiesen werden konnten.

Gemäß den in der EP-A - 0 252 064 dargelegten Erkenntnissen muß eine derartige Vakzine notgedrungen alle Partialantigene enthalten, d.h. ein polyvalenter Impfstoff sein.

Aufgabe der vorliegenden Erfindung ist es, divalente Vakzine, enthaltend nur zwei Antigene, zur Verfügung zu stellen, die im Menschen zur Bildung von Antikörpern führen, die gegen motile Pseudomonas aeruginosa-Keime aller Kombinationen der Partial-Flagella(H)-Antigene $a_0$ bis $a_4$ und b und damit gegen alle klinisch relevanten Serotypen wirken, wobei sie die Motilität und die weitere Verbreitung und Vermehrung der Keime hemmen und die Abtötung durch das menschliche Immunsystem induzieren.

Weiters ist es Aufgabe der vorliegenden Erfindung, Präparationen zur Verfügung zu stellen, die Antikörper gegen diese Antigene enthalten und zur passiven Immunisierung gegen Pseudomonas aeruginosa-Infektionen verwendet werden können.

Diese Aufgabe wird erfindungsgemäß dadurch gelöst, daß die Vakzine eine Kombination eines einzigen Flagella(H)-Antigens vom Serotyp a mit dem Flagella(H)-Antigen vom Serotyp b enthalten.

Es hat sich gezeigt, daß die Immunglobuline, die nach Anwendung der erfindungsgemäßen Impfstoffe gebildet werden, in der Lage sind, an mindestens 95 % aller klinisch relevanten Stämme von Pseudomonas aeruginosa zu binden und deren Motilität zu hemmen. Diese Bindung der Antikörper führt weiters zu einer Opsonisierung und Abtötung der motilen Keime durch die phagozytierenden Zellen des humanen Immunsystems.

Vertreter der erfindungsgemäßen divalenten Vakzine können enthalten:

ein einziges Flagella(H)-Antigen vom Serotyp $a_0 a_2$, welches Antigen aus monomeren Bestandteilen besteht, das die Aminosäuren: Asparaginsäure, Threonin, Serin, Glutaminsäure, Glycin, Alanin, Valin, Isoleucin, Leucin, Tyrosin, Phenylalanin, Lysin und Arginin im Verhältnis 68 : 41 : 37 : 46 : 44 : 73 : 29 : 29 : 37 : 3 : 10 : 16 : 16 enthält und ein Molekulargewicht von 45.900 aufweist;

oder ein einziges Flagella(H)-Antigen vom Serotyp $a_0 a_1 a_2$, welches Antigen aus monomeren Bestandteilen besteht, das die Aminosäuren: Asparaginsäure, Threonin, Serin, Glutaminsäure, Glycin, Alanin, Valin, Isoleucin, Leucin, Tyrosin, Phenylalanin, Lysin und Arginin im Verhältnis 76 : 44 : 40 : 52 : 50 : 81 : 32 : 32 : 41 : 4 : 12 : 20 : 18 enthält und ein Molekulargewicht von 51.250 aufweist;

oder ein einziges Flagella(H)-Antigen vom Serotyp $a_0 a_3$, welches Antigen aus monomeren Bestandteilen besteht, das die Aminosäuren: Asparaginsäure, Threonin, Serin, Glutaminsäure, Glycin, Alanin, Valin, Isoleucin, Leucin, Tyrosin, Phenylalanin, Lysin und Arginin im Verhältnis 69 : 35 : 38 : 44 : 47 : 73 : 30 : 30 : 60 : 3 : 12 : 21 : 16 enthält und ein Molekulargewicht von 46.700 aufweist;

oder ein einziges Flagella(H)-Antigen vom Serotyp $a_0 a_3 a_4$, welches Antigen aus monomeren Bestandteilen besteht, das die Aminosäuren: Asparaginsäure, Threonin, Serin, Glutaminsäure, Glycin, Alanin, Valin, Isoleucin, Leucin, Tyrosin, Phenylalanin, Lysin und Arginin im Verhältnis 64 : 33 : 35 : 42 : 44 : 68 : 29 : 29 : 37 : 3 : 10 : 19 : 15 enthält und ein Molekulargewicht von 43.500 aufweist.

Nach der bevorzugten Ausführungsform enthalten die genannten erfindungsgemäßen Vakzine noch zusätzlich das Flagella(H)-Antigen vom Serotyp b, welches Antigen aus monomeren Bestandteilen besteht, das die Aminosäuren:

Asparaginsäure, Threonin, Serin, Glutaminsäure, Glycin, Alanin, Valin, Isoleucin, Leucin, Tyrosin, Phenylalanin, Lysin und Arginin im Verhältnis 74 : 48 : 48 : 49 : 51 : 91 : 38 : 30 : 43 : 4 : 13 : 18 : 18 enthält und ein Molekulargewicht von 53.050 aufweist.

Die erfindungsgemäßen Vakzine werden hergestellt, indem das bzw. die aus Kulturen von Pseudomonas aeruginosa gewonnene(n), gereinigte(n) Flagella(H)-Antigen(e) sterilfiltriert, gegebenenfalls mit einem Adjuvans, wie $Al(OH)_3$ vermischt, gewünschtenfalls mit einem Präservans, wie Thiomersal, versetzt und in eine galenische Zubereitung formuliert wird (werden).

Die erfindungsgemäßen Immunglobulin-G-hältigen, gegen Pseudomonas aeruginosa-Infektionen wirksamen Präparationen werden hergestellt, indem Plasma von mit dem (den) entsprechenden Flagella(H)-Antigen(en) immunisierten menschlichen Spendern mit Proteinfällungsmitteln, wie Ammoniumsulfat, behandelt, die ausgefällte Immunglobulin G-hältige Fraktion gelöst, gereinigt und zu einer galenischen Zubereitung formuliert wird.

Die Erfindung wird durch nachfolgende Beispiele näher erläutert, wobei die Beispiele 1 und 2 die Herstellung von Vakzinen und das Beispiel 3 die Herstellung von Immunglobulin-G-hältigen Präparationen veranschaulichen.

Beispiel 1:

Vakzine-Formulierung für einen monovalenten Pseudomonas aeruginosa-Flagellenimpfstoff

Eine aus Bakterium Pseudomonas aeruginosa 5940-Kultur gewonnene und in oben beschriebener weise durch Detergensbehandlung, Scheren und chromatographische Behandlung gereinigte Flagellenlösung wurde mit destilliertem pyrogenfreiem Wasser auf eine Proteinkonzentration von 100 $\mu$g/ml eingestellt und durch Zugabe von festem Natriumchlorid und Thiomersal (Endkonzentration 0,9 % NaCl bzw. 0,01 % Thiomersal) isoton gemacht. Anschließend wurde die Lösung durch 0,2 $\mu$m Sterilfilter sterilfiltriert.

Nach einer Proteinbestimmung der sterilfiltrierten Probe wurde die Flagellensuspension mit steriler NaCl/Tiomersallösung (0,9 %/0,01 %) auf 25 $\mu$g/ml Protein eingestellt und anschließend durch Zugabe einer 1 % Aluminiumhydroxidlösung in steriler NaCl/Thiomersallösung auf 20 $\mu$g/ml Protein verdünnt.

Die fertig adjuvantierte Vakzine enthielt pro ml:

20 $\mu$g Flagellenprotein Serotyp $a_0 a_2$

2 mg Aluminiumhydroxid

9 mg NaCl

0,1 mg Thiomersal als Stabilisator

Analog können weitere monovalente Vakzine mit den Pseudomonas aeruginosa-Stämmen 1210 (Serotyp $a_0 a_1 a_2$), 5939 (Serotyp $a_0 a_3$) und 170018 (Serotyp $a_0 a_3 a_4$) hergestellt werden.

Beispiel 2:

Vakzine-Formulierung für einen bivalenten Pseudomonas aeruginosa-Flagellenimpfstoff

Aus Kulturen der Bakterienstämme M-2 und 5939 gewonnene und in oben beschriebener Weise durch Detergensbehandlung und Gelfiltration gereinigte Flagellensuspensionen wurden mit destilliertem, pyrogenfreiem Wasser auf eine Konzentration von 100 $\mu$g/ml Protein eingestellt und durch Zugabe von festem Natriumchlorid und Thiomersal (Endkonzentration 0,9 % NaCl bzw. 0,01 % Thiomersal) isoton gemacht. Anschließend wurden die Lösungen durch 0,2 $\mu$m Filter sterilfiltriert.

Nach einer Proteinbestimmung der sterilfiltrierten Proben wurden die Flagellensuspensionen mit steriler NaCl/Thiomersallösung (0,9 %/0,01 %) verdünnt, wobei die Suspension von M-2 Flagellen auf 50 $\mu$g/ml und

4

die Suspension von 5939 Flagellen auf 25 $\mu$g/ml eingestellt wurden. Beide Suspensionen wurden zu gleichen Volumina vereinigt und durch Zugabe einer 1 % Aluminiumhydroxidlösung in steriler NaCl/Thiomersallösung auf Antigenkonzentrationen von 20 bzw. 10 $\mu$g/ml gebracht.

Die fertig adjuvantierte Vakzine enthielt pro ml:

20 $\mu$g Flagellenprotein Serotyp b

10 $\mu$g Flagellenprotein Serotyp $a_0a_3$

2 mg Aluminiumhydroxid

9 mg NaCl

0,1 mg Thiomersal als Stabilisator

Anstelle der oben beschriebenen Suspension von Flagellen des Pseudomonas-Stammes 5939 können analog Flagellen mit Serotyp $a_0a_3$ aus anderen Stämmen sowie Flagellen mit den Serotypen $a_0a_2$; $a_0a_1a_2$ oder $a_0a_3a_4$ in einer wie oben beschriebenen Mischung mit Flagellen vom Serotyp b verwendet und als Vakzine formuliert werden.

Beispiel 3:

3.1. Immunisierung von Plasmaspendern mit der Flagellenvakzine

50 freiwillige Probanden erhielten je 20 $\mu$g des nach Beispiel 1 aus der Kultur 5940 hergestellten Impfstoffes subcutan. Nach 4 Wochen erhielten 40 Probanden wieder je 20 $\mu$g subcutan und nach weiteren 4 Wochen wurde erneut mit 20 $\mu$g Flagellenantigen immunisiert. Die Bildung von Flagellenantikörpern im Verlauf der Immunisierung wurde mit einem weiter unten beschriebenen Flagellen-ELISA verfolgt. Nach der Immunisierung wurde Plasma von den dreimal immunisierten Probanden gesammelt und gepoolt.

3.2. Herstellung der spezifischen Pseudomonas aeruginosa Flagellen-Immunglobulinpräparation

Die Isolierung der die Antikörper gegen Pseudomonas aeurginosa enthaltenden Immunglobuline erfolgte nach dem in der AT-B - 359.641 beschriebenen Verfahren. Dazu wurde das von den Spendern gewonnene, gepoolte Plasma auf einem pH-Wert von 7,0 bis 7,2 gestellt und auf einer Temperatur von -2°C gehalten. Der Lösung wurden 8 Gew.% Äthanol zugesetzt, wobei ein Niederschlag, der im wesentlichen Fibrinogen enthält, ausgefällt wurde. Nach Abtrennen dieses Niederschlages wurde die Äthanolkonzentration auf 25 Gew.% erhöht und die Temperatur auf -6°C abgesenkt. Der ausfallende Niederschlag, der im wesentlichen aus rohem Immunglobulin besteht, wurde in einem Phosphat-Acetat-Puffer extrahiert und mit 12 Gew.% Äthanol bei einem pH-Wert von 5,3 bei -2°C versetzt. Der Niederschlag (der alpha- und beta-Globulin enthält) wurde verworfen und die Äthanol-Konzentration des Überstandes bei einem pH-Wert von 7,2 und einer Temperatur von -10°C auf 25 Gew.% erhöht, wodurch das Immunglobulin ausgefällt wurde. Die so hergestellte gesammelte pastenförmige gamma-Globulinfraktion wurde erfindungsgemäß in folgender Weise weiterbehandelt:

1 kg der Immun-Globulinpaste wurde in 2 l einer 0,9 % NaCl-Lösung unter Rühren gelöst und dialysiert. Danach wurde die Proteinkonzentration auf 20 g/l sowie die Ionenstärke auf 0,15 gestellt, und bei einem pH-Wert von 6,25 wurden 176 g/l Ammoniumsulfat zugesetzt, der Niederschlag verworfen und dem Überstand weiteres Ammoniumsulfat bis zum Erreichen einer Konzentration von 275 g/l zugegeben und der pH-Wert auf 7,2 eingestellt. Der ausfallende immunglobulinhältige Niederschlag wurde in Wasser gelöst und einer Dialyse gegen Leitungswasser unterworfen. Sodann wurden der Lösung 80 g/l Polyäthylenglykol in Anwesenheit von 150 g/l Glucose bei einem pH-Wert von 6,0 und einer Ionenstärke von 0,15 zugefügt. Der Niederschlag wurde verworfen, und es wurde bei einem pH-Wert von 6,5 bis 6,6 die Polyäthylenglykol-Konzentration auf 95 g/l erhöht. Der neuerlich ausfallende Niederschlag wurde verworfen.

Nun wurde der Gehalt der Lösung an Polyäthylenglykol bei einem pH-Wert von 7,2 auf 180 g/l erhöht, wodurch das von Verunreinigungen freie Immunglobulin ausgefällt wurde. Das Produkt wurde zentrifugiert und durch fünfmaliges Waschen das noch vorhandene Polyäthylenglykol entfernt.

Das so gewonnene intravenös verabreichbare spezifische Pseudomonas-Flagellen-Immunglobulin wurde mit Aqua dest. zu einer 5 % (w/v) Lösung rekonstituiert. Der Titer an spezifisch gegen Flagellen vom Serotyp $a_0a_2$ gerichteten Antikörpern wurde im Flagellen-ELISA bestimmt und wird in Tabelle 2 gezeigt. Hierbei steht der mit 1 : 25.000 angegebene Titer für die 1 : 25.000-Verdünnung der 5 %igen Immunglobulinlösung, die im ELISA-Test noch eine eindeutig nachweisbare positive Rekation ergab.

Gemäß diesem Verfahren wurden auch alle anderen, weiter unten genannten Pseudomonas aeruginosa Flagellen-Immunglobulinpräparationen hergestellt.

Zur Dokumentation der Wirksamkeit der erfindungsgemäßen Vakzine und der erfindungsgemäßen Immunglobulin-G-hältigen, gegen Bakterium Pseudomonas aeruginosa-Infektionen wirksamen Präparationen (Pseudomonas aeruginosa Flagellen-Immunglobulinpräparationen) wird zunächst gezeigt, daß die Flagella-(H)-Antigene (Serotypen b, $b(a_0)$, $a_0a_2$, $a_0a_1a_2$, $a_0a_3$ und $a_0a_3a_4$, die insgesamt alle sechs Partialantigene der klinisch relevanten Pseudomonas-Keime enthalten, beim Menschen zur Bildung von Antikörpern führen.

Zu diesem Zweck wurden Flagellen der oben beschriebenen sechs relevanten Serotypen auf einer SDS-Polyacrylamidgelelektrophorese von eventuell noch anhaftenden, minimalen Verunreinigungen getrennt. Die Flagellenbanden, die das durch das Detergens SDS und Mercaptoäthanol depolymerisierte Flagellin enthalten, wurden bei folgenden Molekulargewichten erhalten: Typ b: 53.050, Typ $a_0$: 52.000, Typ $a_0a_1a_2$: 51.250, Typ $a_0a_2$: 45.900, Typ $a_0a_3a_4$: 43.500, Typ $a_0a_3$: 46.700. Die Molekulargewichte wurden über die Laufstrecken der Standardproteine Carboanhydrase (31.000 MG), Ovalbumin (45.000 MG) und BSA (66.200) berechnet. Die Proteinbanden wurden durch Elektrotransfer aus dett Polyacrylamidgel auf eine Nitrocellulosefolie übertragen. Nachdem freie Proteinbindungsstellen auf der Nitrocellulosefolie durch Absättigen mit Tween® 80 geblockt worden waren, wurde die Folie in einer geeigneten Verdünnung eines Humanserums inkubiert. Dieses Serum wurde von Spendern erhalten, die mit monovalenten Flagellenvakzinen, enthaltend die oben genannten Antigene, mehrmals immunisiert worden waren. Zuvor war durch ELISA-Kontrollen sichergestellt worden, daß die Seren der Spender vor der Immunisierung keine signifikanten Flagellen-Antikörpertiter enthielten.

Die Anwesenheit von flagellenspezifischen Antikörpern nach der Immunisierung konnte durch Bindung dieser Antikörper an die jeweilige Flagellinbande auf der Nitrocellulosefolie nachgewiesen werden, die durch Entwicklung mit einem enzymmarkierten zweiten Antikörper und einem chromogenen Substrat auf der Folie sichtbar gemacht werden kann. In Grenzen läßt dabei die Intensität der Färbung Rückschlüsse auf die Menge an gebundenem spezifischem Antikörper zu.

Die Ergebnisse sind in der Tabelle 1 zusammengefaßt, wobei zur Evaluierung die mit dem homologen Antiserum erzielten Färbeintensitäten "+ +" als gesetzt und die Bindung der Antikörper an Flagellin mit anderen Serotypen in Relation dazu bewertet wurde. War keine Bande auf der Nitrocellulosefolie zu erkennen, wurde in der Tabelle "-" vermerkt.

Tabelle 1

| Kreuzreaktionen von H-Antiseren im Western-blot | | | | | | |
|---|---|---|---|---|---|---|
| Serum gegen H-typ | Antigen auf Western-blot | | | | | |
| | b | $b(a_0)$ | $a_0a_2$ | $a_0a_1a_2$ | $a_0a_3$ | $a_0a_3a_4$ |
| b | + + | + + | (+) | (+) | - | (+) |
| $b(a_0)$ | + + | + + | - | - | - | - |
| $a_0a_2$ | + | + | + + | + | + | + + |
| $a_0a_1a_2$ | + | + | + | + + | + + | + + + |
| $a_0a_3$ | + | + | + | + | + + | + |
| $a_0a_3a_4$ | + | + | + | + | + | + + |

Tabelle 1 ist eine Kreuzreaktivität zu entnehmen, da z.B. Serum gegen ein bestimmtes a-Typ-Flagellin auch gegen andere Antigene vom Typ a reagiert. Es ist sogar ersichtlich, daß Antikörper gegen a-Typ-Flagellin nicht nur an dieses, sondern auch an b- bzw. $b(a_0)$-Typ-Flagellin binden. Die Intensität dieser Bindung ist jedoch meist deutlich schwächer als die bei den verschiedenen a-Typen beobachtete Kreuzreaktion untereinander. Umgekehrt binden Antikörper gegen b- bzw. $b(a_0)$-Typ-Flagellin nicht oder nur sehr schwach an Flagellin vom Typ a.

Zum Nachweis, daß die Flagella(H)-Antigene b, $b(a_0)$, $a_0a_2$, $a_0a_1a_2$, $a_0a_3$ und $a_0a_3a_4$ zur Gewinnung hochtitriger Antikörper bzw. hochtitriger humaner, intravenös verabreichbarer Immunglobulin-G-hältiger Präparationen eingesetzt werden können, wurden im Rahmen der Phase I einer klinischen Studie freiwillige Probanden in regelmäßigen Abständen mehrmals mit den monovalenten Antigenen b, $a_0$, $a_0a_2$, $a_0a_1a_2$, $a_0a_3$ und $a_0a_3a_4$ immunisiert. Von diesen Spendern wurde Plasma gesammelt und nach Serotyp getrennt gepoolt. Daraus wurden nach der in Beispiel 3 beschriebenen Methode Immunglobulin-G-Präparationen für intravenöse Applikation hergestellt. Diese werden im folgenden mit Pseudomonas Flagellen-Immunglobulin (PsH) unter Beifügung des jeweiligen Flagellenserotyps bezeichnet. Zum Beispiel steht PsHb für das aus dem Plasma jener Spender gewonnene Präparat, die mit Flagellen-Vakzine vom Antigen Typ b immunisiert wurden.

Die H-Serotyp-spezifischen Titer wurden mit einem Flagellin-ELISA ermittelt. Hierzu wurden hochgereinigte Flagellenpräparationen 30 min bei Raumtemperatur in einer einprozentigen Lösung von Natriumdodecylsulfat inkubiert, was zu einer Depolymerisierung der Flagellen in Flagellinmonomere führte. Danach wurde die Flagellinlösung über eine Gelfiltrationssäule gegen einen Carbonatpuffer mit einem pH-Wert von 9,6 umgepuffert und nach einer Proteinbestimmung auf eine Konzentration von 0,5 $\mu$g/ml eingestellt. Flagellin aus dieser Lösung wurde dann an feste Träger (Trog von Mikrotiterplatte oder Kamm) gebunden. Nach dem Blocken von freien Proteinbindestellen mit einer 0,05 % Lösung von Tween® 20 in phosphatgepufferter Saline konnten nur noch flagellenspezifische Antikörper an das immobilisierte Flagellin auf dem Träger binden.

Nach mehreren Waschschritten wurden die gebundenen Antikörper mit einem enzymmarkierten zweiten Antikörper (Anti-Human-IgG POD-Konjugat) nachgewiesen und kolorimetrisch quantitativ bestimmt. Die ermittelten Titer sind in der Tabelle 2 angegeben und beziehen sich auf gleiche $\mu$g-Mengen der verschiedenen Flagellenantigene.

Die Titerwerte geben die Verdünnung der 5 %igen Pseudomonas Flagellen-Immunglobulinlösungen an, die im ELISA-Test noch eine eindeutig positive Reaktion ergaben.

Die Menge an immobilisiertem Protein wurde nach Hydrolyse des an jeweils 10 ELISA-Kämmen gebundenen Proteins über Aminosäureanalyse bestimmt, wozu die in der EP-A - 0 252 064 aufgeführten Aminosäurezusammensetzungen der gereinigten Flagellenantigene herangezogen wurden.

Tabelle 2

| ELISA-Titer der Immunglobulinpräparate | | |
|---|---|---|
| H-Serotyp | Stamm | Titer |
| PsH-b | M-2 | 1 : 10.000 |
| PsH-$a_0 a_2$ | 5940 | 1 : 25.000 |
| PsH-$a_0 a_3$ | 5939 | 1 : 30.000 |
| PsH-$a_0 a_1 a_2$ | 1210 | 1 : 25.000 |
| PsH-$a_0 a_3 a_4$ | 170018 | 1 : 35.000 |

Als nächstes wird gezeigt, daß Antikörper aus den so gewonnenen Pseudomonas Immunglobulinpräparaten an Flagellinmoleküle vom homologen Flagellenserotyp binden und daß außerdem die bei Serum von Einzelspendern beobachtete Kreuzreaktivität (Tabelle 1) auch den Hyperimmunglobulinpräparationen zueigen ist.

Hierzu wurden, wie oben beschrieben, Western-blots von Flagellenpräparationen hergestellt. Als erste Antikörper wurden anstelle von Humanseren Lösungen der fünf Hyperimmunglobulinpräparate eingesetzt, die entsprechend dem Flagellen-ELISA auf gleiche Titer eingestellt und dann passend weiterverdünnt wurden. Die weitere Entwicklung und Auswertung der Blots erfolgte, wie bei Tabelle 1 beschrieben.

Die Ergebnisse sind in der Tabelle 3 zusammengefaßt.

Tabelle 3

| Kreuzreaktivität von flagellenspezifischen Pseudomonas-Immunglobulinpräparaten | | | | | | |
|---|---|---|---|---|---|---|
| AK | Antigen auf Western-blot | | | | | |
| | b | $b(a_0)$ | $a_0 a_2$ | $a_0 a_1 a_2$ | $a_0 a_3$ | $a_0 a_3 a_4$ |
| PsHb | + + | + + | (+) | (+) | (+) | + |
| PsHa$_0 a_2$ | + | + | + + | + + | + + | + + + |
| PsHa$_0 a_1 a_2$ | + | + | + | + + | + + | + + + |
| PsHa$_0 a_3$ | + | + | + + | + + | + + | + + |
| PsHa$_0 a_3 a_4$ | + | + | + + | + | + | + + |

Wie schon mit Seren von Einzelspendern gezeigt, läßt sich auch bei den untersuchten Immunglobulinpräparationen eine starke Kreuzreaktivität aller gegen a-Typ-Flagellen gerichteter Antikörper mit Flagellinmolekülen aller a-Subklassenzusammensetzungen beobachten. Ebenso ist evident, daß eine (schwächere) Bindung von gegen a-Typ Flagellen gerichteten Antikörpern an b-Typ Flagellin erfolgen kann. Relativ schwach, aber noch deutlich nachweisbar, ist die Bindung von b-Typ Antikörpern an a-Typ Flagellin.

Die Wirksamkeit der erfindungsgemäßen Antikörper auf motile Pseudomonas aeruginosa-Keime kann auf eine Hemmung ihrer Motilität - und damit der Ausbreitung des Infektionsherdes - zurückgeführt werden.

Diese Motilitätshemmung kann auf Weichagar-Schwärmplatten demonstriert werden. Dazu wurde eine Filterpapierrondelle mit 20 $\mu$l einer 0,5 %igen Lösung von PsH in HBSSG getränkt. Diese wurde auf ein Inokulum von $10^5$ (10h5) Keimen in 10 $\mu$l aufgelegt, nachdem die Keimsuspension in den Weichagar eingedrungen war. Der Grad der Hemmung der Motilität wurde über eine auf derselben Platte befindlichen Kontrolle bestimmt, bei der auf das Inokulum eine in Hank's Balanced Salt Solution mit Gelatine (HBSSG) getränkte Filterpapierrondelle aufgelegt wurde. Ähnlich wie im Western-blot konnten Kreuzreaktionen zwischen Antikörpern gegen verschiedene a-Serotyp Flagellen und allen untersuchten Keimen mit Flagellen verschiedener a-Subtypzusammensetzung festgestellt werden. Auch die im Western-blot beobachtete Kreuzreaktion zwischen a-Typ Antikörpern und b-Flagellen war zu beobachten. Deutlicher als im Western-blot erschien die Kreuzreaktion zwischen b-Typ Antikörpern und a-Typ Flagellen aller Subklassen.

Die Ergebnisse sind in Tabelle 4 zusammengefaßt, wobei die Durchmesser der Schwärmzonen von Keimen, die mit Antikörpern inkubiert wurden, mit auf derselben Platte befindlichen antikörperfreien Kontrollen verglichen wurden: " + + " bezeichnet einen Durchmesser der Schwärmzone, der mindestens 40 % kleiner ist als jener der Kontrolle; " + ": Durchmesser mindestens 20 %, aber höchstens 40 % kleiner als die Kontrolle.

Tabelle 4

| Kreuzreaktionen von H-Antikörpern im Motilitäts-Hemmtest | | | | | | |
|---|---|---|---|---|---|---|
| AK gegen H-Typ | H-Antigen des Keims auf der Schwärmplatte | | | | | |
| | b | b($a_0$) | $a_0 a_1 a_2$ | $a_0 a_2$ | $a_0 a_3$ | $a_0 a_3 a_4$ |
| b | + + | + + | + + | + | + | + + |
| $a_0 a_1 a_2$ | + | + | + | + | + | + |
| $a_0 a_2$ | + | + | + + | + + | + + | + |
| $a_0 a_3$ | + | + | + | + + | + + | + + |
| $a_0 a_3 a_4$ | + + | + | + | + + | + | + + |

Die Ergebnisse der Tabelle 4 zeigen, daß die mit Flagella(H)-Antigenen gewonnenen erfindungsgemäßen Antikörper die Motilität von Pseudomonas-Bakterienstämmen hemmen.

Durch die Hemmung der Motilität der in einem Infektionsherd befindlichen Bakterien wird zwar deren weitere Ausbreitung nach der Kolonisierung im Körper des Patienten verhindert, die im Herd befindlichen Bakterien bleiben jedoch weiterhin in der Lage, den Organismus des Patienten durch Abgabe z.B. von Toxinen, Proteasen, etc. zu belasten. Ein echter Schutz gegen Infektion ist deshalb nur dann gewährleistet, wenn die bei der aktiven Immunisierung gebildeten Antikörper zu einer schnellen Abtötung der eingedrungenen Bakterien führen.

Im folgenden wird der Nachweis geliefert, daß Flagella(H)-Antikörper Phagocytose und intrazelluläre Abtötung des homologen Pseudomonas-Bakterienstammes induzieren.

Zur Durchführung der Phagocytoseexperimente wurden Keime der Stämme M-2 (b), WR-5 ($a_0 a_2$), 1210 ($a_0 a_1 a_2$), 5939 ($a_0 a_3$) und 170018 ($a_0 a_3 a_4$) in Luria Broth Medium angezüchtet und kurz vor Beginn der stationären Wachstumsphase in HBSSG auf eine Lebendkeimzahl von 8 . $10^8$ (8.10h8) Keime pro ml verdünnt.

Von allen 5 %igen Flagellen-Immunglobulinpräparationen wurde eine 1 : 18-, 1 : 72- und 1 : 288-Verdünnung in HBSSG hergestellt. Je 450 $\mu$l dieser Verdünnungen wurden mit 50 $\mu$l Keimsuspension und 4,5 ml HBSSG 2 h auf Eis inkubiert. Bei dieser Inkubation erfolgte Bindung von spezifischen Antikörpern an Bakterienzellen.

Als phagocytierende Zellen wurden humane Granulocyten verwendet. Diese wurden aus frisch isolierten "Buffy Coats" gewonnen. Die Buffy Coats wurden durch Unterschichten mit Saline-Dextrose-Dextranlösung vom Großteil der Erythrocyten befreit. Daran wurden mehrere Zentrifugationsschritte angeschlossen, durch die restliche Erythrocyten und Thrombocyten weitgehend entfernt wurden. Nach Waschen in Saline wurde die Zellzahl im Coulter® Zellzählgerät bestimmt und auf 1 . $10^7$ (1.10h7) Zellen/ml in HBSSG eingestellt. Von diesen Zellsuspensionen wurde ein Differentialblutbild erstellt. Dabei sollte das Verhältnis von Granulocyten zu Lymphocyten im Normbereich von etwa 6 : 4 liegen. Der Gehalt an Monocyten liegt normalerweise zwischen 4 und 8 %. Zellisolate, die diese Kriterien erfüllen, können im Phagocytosetest eingesetzt werden.

Als Komplementquelle wurde absorbiertes Humanserum (ABA) aus einem Pool von Spendern mit der Blutgruppe AB eingesetzt. Dieses Serum wurde zuvor an alle oben genannten Keime adsorbiert, wobei 1 ml Serum pro Keim 5 mal mit $5 \times 10^9$ (5x10h9) Keimen 0,5 h auf Eis inkubiert wurde. Auf jede Inkubation folgte ein Zentrifugationsschritt von 5 min bei 8.000 g. Nach der letzten Inkubation wurde zusätzlich 15 min bei 14.000 g zentrifugiert. Nach der Absorption wurde der Komplementgehalt des Serums in C'H50-Einheiten ausgetestet. Dieser lag 15 % unter dem des Ausgangsserums. Das so gewonnene ABA-Serum wurde in Hank's Puffer 1 : 50 vorverdünnt und im Phagocytosetest eingesetzt.

Zum Phagocytosetestansatz wurden 100 $\mu$l der Granulocytensuspension, 60 $\mu$l der Keimsuspension und 40 $\mu$l des absorbierten AB-Serums im Trog einer Mikrotiterplatte gemischt. Sofort nach dem Mischen und nach 1 h Inkubation bei 37°C wurden 25 $\mu$l aus dem Testansatz entnommen und in 5 ml bidest. Wasser verdünnt. Dabei wurden die Granulocyten osmotisch lysiert und darin enthaltene Bakterienzellen freigesetzt. 25 $\mu$l dieser Suspension wurden zur Bestimmung der Lebendkeimzahl ausplattiert. Die Abtötungsrate wurde mit den Koloniezahlen des 0- und 60 min-Wertes nach folgender Formel berechnet:

$$\% \; \text{Abtötung} = 1 - \frac{\dfrac{\text{Zahl } t = 60}{\text{Kontrolle}}}{\text{Zahl } t = 0} \times 100$$

Zur Berechnung des Kontrollwertes dienten die $t = 0$ und $t = 60$ min-Lebendkeimwerte, die von einer Position im Test erhalten wurden, bei der anstelle der antikörperbeladenen Keime unbeladene Keime eingesetzt wurden. Der Quotient aus den damit erhaltenen 60- und 0-min-Werten wurde als Kontrolle in die obige Formel eingesetzt.

Die Ergebnisse der Phagocytosetests sind in Tabelle 5 zusammengefaßt.

Tabelle 5

| Abtötung von Pseudomonas aeruginosa nach Opsonisierung mit Flagellenantikörpern durch Humangranulocyten | | | | | | |
|---|---|---|---|---|---|---|
| AK gegen H-Typ | | H-Antigen des Keims im Phagocytosetest | | | | |
| | | b | $a_0 a_1 a_2$ | $a_0 a_2$ | $a_0 a_3$ | $a_0 a_3 a_4$ |
| PsHb | 1: | 30 | hoch* | hoch | 40 | 27 |
| PsHa$_0$a$_2$ | 1: | 60 | 102 | 120 | 86 | 65 |
| PsHa$_0$a$_3$ | 1: | 78 | 105 | hoch | 108 | 84 |
| PsHa$_0$a$_1$a$_2$ | 1: | 60 | 200 | hoch | 125 | 72 |
| PsHa$_0$a$_3$a$_4$ | 1: | 78 | hoch | 190 | 80 | 78 |

*: Bei allen Antikörperkonzentrationen wurden weniger als 50 % der eingesetzten Keime abgetötet

Die in der Tabelle angegebenen Titerwerte zeigen die Konzentration der H-typspezifischen Flagellen-Immunglobulinpräparation, die bei der Opsonisierung von $4 \times 10^8$ (4x10h8) Keimen in einem 5 ml-Ansatz eingestellt werden mußte, um im Phagocytosetest eine Abtötung von 50 % der eingesetzten Keime zu erreichen. Berechnet wurden diese Phagocytosetiter als Quotient aus den im Flagellen-ELISA ermittelten Absolutwerten der homologen Antikörpertiter und den im Phagocytoseversuch bestimmten Verdünnungsfaktoren, bei denen 50 % der eingesetzten Keime durch das jeweilige (homologe oder heterologe) Flagellen-Immunglobulin abgetötet wurden.

In Tabelle 5 ist ersichtlich, daß Pseudomonas-Flagellen-Immunglobulinpräparate Keime mit homologen und heterologen Flagellenserotypen opsonisieren. Hierbei ist besonders auffällig die Kreuzreaktion aller PsHa-Präparate mit allen a-Typ- und b-Typ-Keimen. Besonders niedrige Konzentrationen von PsHa$_0$a$_2$ induzieren die Phagocytose von Keimen mit a- und b-Typ-Flagellen. Zur Abtötung von b-Typ-Keimen sind höhere Konzentrationen von PsHa- als von PsHb-Präparaten erforderlich.

Diese Ergebnisse zeigen, daß Antikörper mit $a_0a_2$-Spezifität Schutzwirkung gegen Keime mit allen klinisch relevanten Flagellenserotypen haben und daß diese Schutzwirkung in einer Mischung, bestehend aus $a_0a_2$-Antikörpern und b-Antikörpern in bezug auf Keime mit b-Typ-Flagellen verstärkt sein muß.

Es war bisher nicht bekannt, ob Antikörper, die aus Plasma von mit hochgereinigten Flagellenantigenen immunisierten Spendern isoliert wurden, eine protektive Wirkung gegen Infektionen mit Pseudomonas aeruginosa besitzen. Mit dem oben beschriebenen Phagocytosetest wurde dieser Nachweis für PsH-Präparate mit Spezifität für alle klinisch relevanten H-Serotypen erbracht.

In der folgenden Tabelle 6 ist die Wirksamkeit des erfindungsgemäßen monovalenten Antikörperpräparates $PsHa_0a_2$ und die Wirkung der erfindungsgemäßen bivalenten Präparate $PsHb/a_0a_2$, $PsHb/a_0a_1a_2$, $PsHb/a_0a_3$ und $PsHb/a_0a_3a_4$, die nach der in Beispiel 2 beschriebenen Methode hergestellt wurden, angegeben.

Tabelle 6

| Wirksamkeit von therapeutischen Pseudomonas-Immunglobulinpräparaten im Phagocytosetest | | | | | | | |
|---|---|---|---|---|---|---|---|
| AK gegen H-Typ | | H-Antigen des Keims im Phagocytosetest | | | | | |
| | | b | $a_0a_1a_2$ | $a_0a_2$ | $a_0a_3$ | $a_0a_3a_4$ | 0 |
| $PsHa_0a_2$ | 1: | 60 | 102 | 120 | 86 | 65 | 87 |
| Standard | 1: | 3 | 19 | 31 | 28 | 9 | 18 |
| $PsHb/a_0a_2$ | 1: | 6 | 24 | 30 | 80 | 20 | 32 |
| $PsHb/a_0a_1a_2$ | 1: | 5 | 28 | 59 | 32 | 13 | 27 |
| $PsHb/a_0a_3$ | 1: | 11 | 23 | 32 | 25 | 7 | 20 |
| $PsHb/a_0a_3a_4$ | 1: | 19 | 34 | 15 | 54 | 6 | 26 |

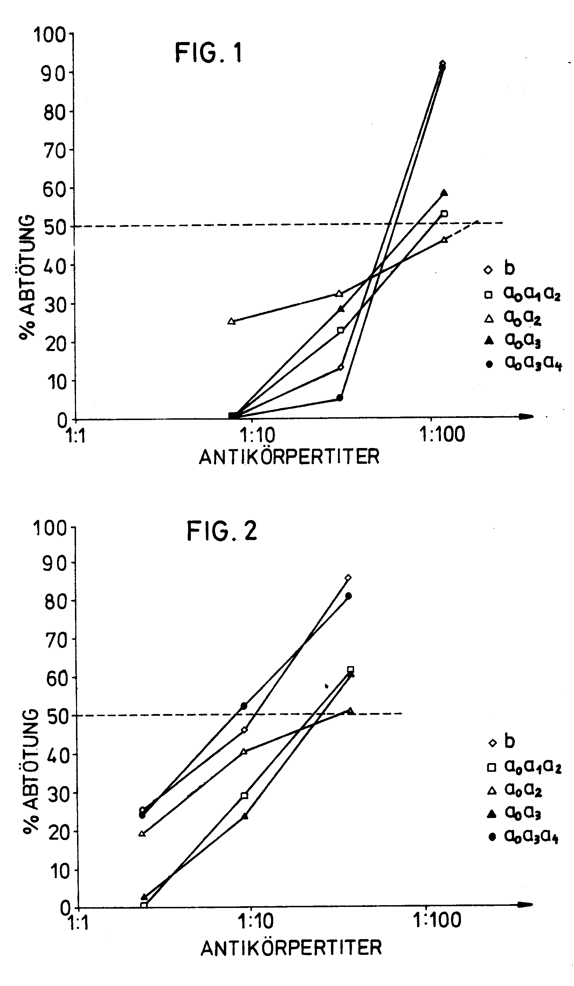

In Tabelle 6 ist zunächst die Wirksamkeit des $PsHa_0a_2$-Präparates im Phagocytosetest angegeben. Hierbei stehen die Titerangaben für jene Konzentration des Immunglobulins, die bei der Beladung der Keime eingestellt werden muß, um anschließend im Phagocytosetest eine 50 %ige Abtötung der Keime zu erreichen. In Fig. 1 ist dargestellt, wie diese Titer ermittelt wurden: 450 $\mu$l von 1 : 18-, 1 : 72- und 1 : 288-Vorverdünnungen der 5 %igen $PsHa_0a_2$-Lösung werden mit 50 $\mu$l einer Suspension von 8 x $10^8$ ($8x10h8$) 5940-Keimen/ml und mit 4,5 ml Hank's-Puffer mit Gelatine versetzt. Dies resultiert in Endverdünnungen von 1 : 200, 1 : 800 und 1 : 3200. Der im Flagellen-ELISA ermittelte Titer von $PsHa_0a_2$ liegt bei 1 : 25.000. Damit berechnen sich die Konzentrationen der Flagellenantikörper während der Beladung der Bakterien mit 1 : 125, 1 : 31,3 und 1 : 7,8. In der Fig. 1 ist die im Phagocytosetest erzielte Abtötung der Keime gegen diese Immunglobulinkonzentrationen aufgetragen. Aus diesen Abtötungskurven lassen sich die in der Tabelle angegebenen Konzentrationen von Immunglobulin ermitteln, bei denen 50 % des untersuchten Keimes abgetötet werden.

Um die Wirksamkeit der $PsHa_0a_2$-Präparation abschätzen zu können, dient als Vergleich ein Standard, der eine Mischung aus allen fünf in Tabelle 6 angeführten PsH-Präparationen darstellt, wobei alle Einzelkomponenten auf einen ELISA-Titer von 1 : 2.000 eingestellt wurden.

Tabelle 6 zeigt weiters die Wirksamkeit von Mischungen aus PsHb und den Präparationen $PsHa_0a_2$ bzw. $PsHa_0a_1a_2$ bzw. $PsHa_0a_3$ bzw. $PsHa_0a_3a_4$. Diese Mischungen wurden auf einen anti-b-Titer von ca. 1 : 7.500 und einen homologen anti-a-Titer von derselben Höhe eingestellt. Tabelle 6 zeigt die Phagocytositer, die mit diesen Immunglobulinmischungen für Pseudomonas aeruginosa-Stämme mit Flagellenepitopen aller klinisch relevanten Serotypen ermittelt wurden.

Diese Titer stehen für die Konzentration der Immunglobulinmischung, die ausgehend von einem Titer 1 : 7.500 für beide Komponenten bei der Beladung der Keime eingestellt werden mußte, um anschließend im Phagocytosetest eine Abtötung von 50 % der eingesetzten Keime zu erreichen.

Fig. 2 kann entnommen werden, wie diese Titer für die Mischung von PsHb und $PsHa_0a_3$ ermittelt wurden: Eine Mischung von 1 ml PsHb und 0,33 ml $PsHa_0a_3$, resultierend in einem b und $a_0a_3$-Titer von 1 : 7.500 wurde, wie oben beschrieben, weiterverdünnt und im Phagocytosetest eingesetzt. In Fig. 2 ist die im Phagocytosetest erzielte Abtötung gegen die bei der Beladung der Bakterien vorhandenen Antikörpertiter für die Einzelkomponenten b und $a_0a_3$ aufgetragen.

Tabelle 6 zeigt, daß das $PsHa_0a_2$-Präparat zwar gegen alle relevanten Serotypen wirkt, seine Wirksamkeit jedoch nicht so ausgeprägt ist wie jene des Standards. Demgegenüber weisen Mischungen, bestehend aus PsHb und einem der Präparate $PsHa_0a_2$, $PsHa_0a_1a_2$, $PsHa_0a_3$ oder $PsHa_0a_3a_4$, eine Wirksamkeit auf, die jener der Standardpräparation nahezu gleicht und die Wirksamkeit von Einzelkomponentenpräparaten (vgl. Tabelle 5) deutlich übertreffen. Zur Verdeutlichung ist in der Spalte unter 0 ein Durchschnittstiter für die verschiedenen Präparate bzw. Mischungen angegeben.

Immunglobulinpräparationen, die durch Mischen aus Plasma von Spendern gewonnen wurden, die mit Flagellenantigen des Serotyps b und einem der vier untersuchten a-Subtypkombinationen immunisiert wurden, sind demnach in der Lage, die Abtötung von Keimen mit Flagellen aller klinisch relevanten Serotypen zu induzieren. Naturgemäß sind nach den oben beschriebenen Ergebnissen auch erfindungsgemäße bivalente Vakzine, bestehend aus dem Flagellenantigen b und einem der Antigene $a_0 a_2$, $a_0 a_1 a_2$, $a_0 a_3$ oder $a_0 a_3 a_4$, oder ein nach deren Anwendung gewonnenes erfindungsgemäßes Immunglobulinpräparat in der Lage, gegen Infektionen mit flagellentragenden Pseudomonas aeruginosa-Bakterien zu schützen.

Der direkte Nachweis der Schutzwirkung von Antigenen, die zu aktiver Immunisierung verwendet werden sollen, kann nur im Tiermodell geführt werden und ist nachfolgend beschrieben.

Um die Situationen zu simulieren, die Patienten für Pseudomonas-Infektionen prädisponieren, wurden zwei in der Literatur beschriebene Tiermodelle verwendet: das burned mouse Modell (Stieritz, D.D. and I.A. Holder (1975) J. Infect. Dis. 131, 688-691) und das Endoxan Modell (Cryz, S.J.Jr., Fürer, E., Germanier, R. (1983), Infect. Immunity 40, 659-664).

Für beide Modelle wurde die $LD_{50}$-Dosis von Pseudomonas aeruginosa in nicht immunisierten Tieren ermittelt, die im Falle des Endoxan Modelles immunsupprimiert wurden; beim burned mouse Modell wurde eine Verbrennung definierter Größe gesetzt. Nach der Immunisierung mit den in Tabelle 7 angeführten Flagella(H)-Antigenen wurden Tiere mit Verbrennungen bzw. immunsupprimierte Tiere mit einem Vielfachen der vorher ermittelten $LD_{50}$-Keimzahl des homologen Pseudomonas-Stammes infiziert. Tabelle 7 zeigt die Vielfachen der $LD_{50}$-Keimzahlen, die von 100 % der mit der angegebenen Konzentration von Antigen immunisierten Tiere im Endoxan- und im burned mouse-Modell toleriert werden. Die Ergebnisse dokumentieren die Wirksamkeit einer bivalenten Flagellenvakzine im normalen und immunsupprimierten Tier.

Tabelle 7

Aktiver Schutzversuch mit Pseudomonas aeruginosa-
Flagellen-Vakzine

| Antigen | Menge/Maus adjuvantiert mit Al(OH)$_3$ | Immunisierung Tage vor Challenge | Endoxan-modell Schutz-faktor (x LD$_{50}$) | | Burned-mouse-Modell Schutzfaktor (x LD$_{50}$) | |
|---|---|---|---|---|---|---|
| b | 3 µg | 14 | b | $3 \times 10^1$ | $> 10^4$ | |
| $a_0 a_3$ | 1,5 µg | 14 | $a_0 a_3$ | $2 \times 10^1$ | $10^3$ | |
| b/$a_0 a_3$ | 3/1,5 µg | 14 | b | $10^2$ | $> 10^4$ | b |
| | | | $a_0 a_2$ | $4 \times 10^1$ | $10^3$ | $a_0 a_1 a_2$ |
| | | | $a_0 a_1 a_2$ | $2 \times 10^1$ | $10^4$ | $a_0 a_2$ |
| | | | $a_0 a_3$ | $10^2$ | $10^4$ | $a_0 a_3$ |
| | | | $a_0 a_3 a_4$ | $10^2$ | $> 10^4$ | $a_0 a_3 a_4$ |

**Patentansprüche**

1. Gegen Pseudomonas aeruginosa-Infektionen wirksame divalente Vakzine, deren Wirksamkeit sich gegen alle Kombinationen der Partial-Flagella(H)-Antigene $a_0$, $a_1$, $a_2$, $a_3$, $a_4$ und b erstreckt, dadurch gekennzeichnet, daß sie eine Kombination eines einzigen Flagella(H)-Antigens vom Serotyp a mit dem Flagella(H)-Antigen vom Serotyp b enthalten.

2. Immunglobulin G-hältige Präparationen, gewonnen aus Plasma von mit divalenten Vakzinen gemäß Anspruch 1 immunisierten Spendern, die die Motilität von Pseudomonas aeruginosa-Keimen hemmen, die Phagozytose dieser Keime verbessern und die Abtötung opsonisierter Keime herbeiführen.

3. Divalente Vakzine nach Anspruch 1, dadurch gekennzeichnet, daß sie das Flagella(H)-Antigen vom H-Serotyp $a_0 a_2$ enthalten, welches Antigen aus monomeren Bestandteilen besteht, das die Aminosäuren: Asparaginsäure, Threonin, Serin, Glutaminsäure, Glycin, Alanin, Valin, Isoleucin, Leucin, Tyrosin, Phenylalanin, Lysin und Arginin im Verhältnis von 68 : 41 : 37 : 46 : 44 : 73 : 29 : 29 : 37 : 3 : 10 : 16 : 16 enthält und ein Molekulargewicht von 45.900 aufweist.

4. Divalente Vakzine nach Anspruch 1, dadurch gekennzeichnet, daß sie das Flagella(H)-Antigen vom H-Serotyp $a_0 a_1 a_2$ enthalten, welches Antigen aus monomeren Bestandteilen besteht, das die Aminosäuren: Asparaginsäure, Threonin, Serin, Glutaminsäure, Glycin, Alanin, Valin, Isoleucin, Leucin, Tyrosin, Phenylalanin, Lysin und Arginin im Verhältnis von 76 : 44 : 40 : 52 : 50 : 81 : 32 : 32 : 41 : 4 : 12 : 20 : 18 enthält und ein Molekulargewicht von 51.250 aufweist.

5. Divalente Vakzine nach Anspruch 1, dadurch gekennzeichnet, daß sie das Flagella(H)-Antigen vom H-Serotyp $a_0 a_3$ enthalten, welches Antigen aus monomeren Bestandteilen besteht, das die Aminosäuren: Asparaginsäure, Threonin, Serin, Glutaminsäure, Glycin, Alanin, Valin, Isoleucin, Leucin, Tyrosin, Phenylalanin, Lysin und Arginin im Verhältnis von 69 : 35 : 38 : 44 : 47 : 73 : 30 : 30 : 60 : 3 : 12 : 21 : 16 enthält und ein Molekulargewicht von 46.700 aufweist.

6. Divalente Vakzine nach Anspruch 1, dadurch gekennzeichnet, daß sie das Flagella(H)-Antigen vom H-Serotyp $a_0 a_3 a_4$ enthalten, welches Antigen aus monomeren Bestandteilen besteht, das die Aminosäuren: Asparaginsäure, Threonin, Serin, Glutaminsäure, Glycin, Alanin, Valin, Isoleucin, Leucin, Tyrosin, Phenylalanin, Lysin und Arginin im Verhältnis von 64 : 33 : 35 : 42 : 44 : 68 : 29 : 29 : 37 : 3 : 10 : 19 : 15 enthält und ein Molekulargewicht von 43.500 aufweist.

7. Divalente Vakzine nach einem der Ansprüche 3 bis 6, dadurch gekennzeichnet, daß sie zusätzlich noch das Flagella(H)-Antigen vom H-Serotyp b enthalten, welches Antigen aus monomeren Bestandteilen besteht, das die Aminosäuren: Asparaginsäure, Threonin, Serin, Glutaminsäure, Glycin, Alanin, Valin, Isoleucin, Leucin, Tyrosin, Phenylalanin, Lysin und Arginin im Verhältnis von 74 : 48 : 48 : 49 : 51 : 91 : 38 : 30 : 43 : 4 : 13 : 18 : 18 enthält und ein Molekulargewicht von 53.050 aufweist.

8. Verfahren zur Herstellung von divalenten Vakzinen gemäß den Ansprüchen 1 und 3 bis 7, dadurch gekennzeichnet, daß das bzw. die aus Kulturen von Pseudomonas aeruginosa gewonnene(n), gereinigte(n) Flagella(H)-Antigen(e) sterilfiltriert, gegebenenfalls mit einem Adjuvans, wie $Al(OH)_3$ vermischt, gewünschtenfalls mit einem Präservans, wie Thiomersal, versetzt und in eine galenische Zubereitung formuliert wird (werden).

9. Verfahren zur Herstellung von Immunglobulin G-hältigen, gegen Bakterium Pseudomonas aeruginosa-Infektionen wirksamen Präparationen gemäß Anspruch 2, dadurch gekennzeichnet, daß Plasma von mit dem (den) entsprechenden Flagella(H)-Antigen(en) immunisierten menschlichen Spendern mit Proteinfällungsmitteln, wie Ammoniumsulfat, behandelt, die ausgefällte Immunglobulin G-hältige Fraktion gelöst, gereinigt und zu einer galenischen Zubereitung formuliert wird.

**Claims**

1. Bivalent vaccines active against Pseudomonas aeruginosa infections, whose activity is directed against any combination of the partial flagellar (H) antigens $a_0$, $a_1$, $a_2$, $a_3$, $a_4$ and b, characterized in that they contain a combination of a single flagellar (H) antigen of serotype a with a flagellar (H) antigen of

serotype $\underline{b}$.

2. Immunoglobulin G-containing preparations obtained from plasma of donors immunized with bivalent vaccines according to claim 1, which preparations inhibit the motility of Pseudomonas aeruginosa pathogens, enhance the phagocytosis of these pathogens and induce the killing of opsonized pathogens.

3. Bivalent vaccines according to claim 1, characterized in that they contain flagellar (H) antigen of H-serotype $a_0a_2$, which antigen consists of monomeric components, contains the aminoacids: aspartic acid, threonine, serine, glutamic acid, glycine, alanine, valine, isoleucine, leucine, tyrosine, phenylalanine, lysine and arginine at a ratio of 68 : 41 : 37 : 46 : 44 : 73 : 29 : 29 : 37 : 3 : 10 : 16 : 16, and has a molecular weight of 45,900.

4. Bivalent vaccines according to claim 1, characterized in that they contain flagellar (H) antigen of H-serotype $a_0a_1a_2$, which antigen consists of monomeric components, contains the aminoacids: aspartic acid, threonine, serine, glutamic acid, glycine, alanine, valine, isoleucine, leucine, tyrosine, phenylalanine, lysine and arginine at a ratio of 76 : 44 : 40 : 52 : 50 : 81 : 32 : 32 : 41 : 4 : 12 : 20 : 18, and has a molecular weight of 51,250.

5. Bivalent vaccines according to claim 1, characterized in that they contain flagellar (H) antigen of H-serotype $a_0a_3$, which antigen consists of monomeric components, contains the aminoacids: aspartic acid, threonine, serine, glutamic acid, glycine, alanine, valine, isoleucine, leucine, tyrosine, phenylalanine, lysine and arginine at a ratio of 69 : 35 : 38 : 44 : 47 : 73 : 30 : 30 : 60 : 3 : 12 : 21 : 16, and has a molecular weight of 46,700.

6. Bivalent vaccines according to claim 1, characterized in that they contain flagellar (H) antigen of H-serotype $a_0a_3a_4$, which antigen consists of monomeric components, contains the aminoacids: aspartic acid, threonine, serine, glutamic acid, glycine, alanine, valine, isoleucine, leucine, tyrosine, phenylalanine, lysine and arginine at a ratio of 64 : 33 : 35 : 42 : 44 : 68 : 29 : 29 : 37 : 3 : 10 : 19 : 15, and has a molecular weight of 43,500.

7. Bivalent vaccines according to any one of claims 3 to 6, characterized in that they additionally contain flagellar (H) antigen of H-serotype $\underline{b}$, which antigen consists of monomeric components, contains the aminoacids: aspartic acid, threonine, serine, glutamic acid, glycine, alanine, valine, isoleucine, leucine, tyrosine, phenylalanine, lysine and arginine at a ratio of 74 : 48 : 48 : 49 : 51 : 91 : 38 : 30 : 43 : 4 : 13 : 18 : 18, and has a molecular weight of 53,050.

8. A method of producing bivalent vaccines according to claims 1 and 3 to 7, characterized in that the purified flagellar (H) antigen(s) obtained from cultures of Pseudomonas aeruginosa is/are sterilely filtered, optionally mixed with an adjuvant, such as $Al(OH)_3$, if desired, admixed with a preservant, such as thiomersalate, and formulated into a galenic preparation.

9. A method of producing immunoglobulin G-containing preparations active against bacterium Pseudomonas aeruginosa infections according to claim 2, characterized in that plasma of human donors immunized with the respective flagellar (H) antigen(s) is treated with protein precipitating agents, such as ammonium sulfate, the precipitated immunoglobulin G-containing fraction is dissolved, purified and formulated into a galenic preparation.

**Revendications**

1. Vaccins bivalents actifs contre les infections par Pseudomonas aeruginosa, dont l'activité est dirigé contre l'ensemble des combinaisons des antigènes flagella(H) partiels $a_0$, $a_1$, $a_2$, $a_3$, $a_4$ et $\underline{b}$, caractérisés en ce qu'ils contiennent une combinaison d'un seul antigène flagella(H) du sérotype $\underline{a}$ avec l'antigène flagella(H) du sérotype $\underline{b}$.

2. Préparations contenant de l'immunoglobuline G obtenues à partir du plasma de donateurs immunisés avec les vaccins bivalents selon la revendication 1, lesquelles préparations inhibent la motilité des germes de Pseudomonas aeruginosa, améliorent le phagocytose desdits germes et induisent la déstruction des germes opsonisés.

3. Vaccins bivalents selon la revendication 1, caractérisés en ce qu'ils contiennent l'antigène flagella(H) du sérotype H $a_0a_2$, lequel antigène est constitué de composants monomères, contient les aminoacides: l'acide aspartique, la thréonine, la sérine, l'acide glutamique, la glycine, l'alanine, la valine, l'isoleucine, la leucine, la tyrosine, la phénylalanine, la lysine et l'arginine dans les rapports de 68 : 41 : 37 : 46 : 44 : 73 : 29 : 29 : 37 : 3 : 10 : 16 : 16, et a un poids moléculaire de 45.900.

4. Vaccins bivalents selon la revendication 1, caractérisés en ce qu'ils contiennent l'antigène flagella(H) du sérotype H $a_0a_1a_2$, lequel antigène est constitué de composants monomères, contient les aminoacides: l'acide aspartique, la thréonine, la sérine, l'acide glutamique, la glycine, l'alanine, la valine, l'isoleucine, la leucine, la tyrosine, la phénylalanine, la lysine et l'arginine dans les rapports de 76 : 44 : 40 : 52 : 50 : 81 : 32 : 32 : 41 : 4 : 12 : 20 : 18, et a un poids moléculaire de 51.250.

5. Vaccins bivalents selon la revendication 1, caractérisés en ce qu'ils contiennent l'antigène flagella(H) du sérotype H $a_0a_3$, lequel antigène est constitué de composants monomères, contient les aminoacides: l'acide aspartique, la thréonine, la sérine, l'acide glutamique, la glycine, l'alanine, la valine, l'isoleucine, la leucine, la tyrosine, la phénylalanine, la lysine et l'arginine dans les rapports de 69 : 35 : 38 : 44 : 47 : 73 : 30 : 30 : 60 : 3 : 12 : 21 : 16, et a un poids moléculaire de 46.700.

6. Vaccins bivalents selon la revendication 1, caractérisés en ce qu'ils contiennent l'antigène flagella(H) du sérotype H $a_0a_3a_4$, lequel antigène est constitué de composants monomères, contient les aminoacides: l'acide aspartique, la thréonine, la sérine, l'acide glutamique, la glycine, l'alanine, la valine, l'isoleucine, la leucine, la tyrosine, la phénylalanine, la lysine et l'arginine dans les rapports de 64 : 33 : 35 : 42 : 44 : 68 : 29 : 29 : 37 : 3 : 10 : 19 : 15, et a un poids moléculaire de 43.500.

7. Vaccins bivalents selon l'une quelconque des revendications 3 à 6, caractérisés en ce qu'ils contiennent de plus l'antigène flagella(H) du sérotype H b, lequel antigène est constitué de composants monomères, contient les aminoacides: l'acide aspartique, la thréonine, la sérine, l'acide glutamique, la glycine, l'alanine, la valine, l'isoleucine, la leucine, la tyrosine, la phénylalanine, la lysine et l'arginine dans les rapports de 74 : 48 : 48 : 49 : 51 : 91 : 38 : 30 : 43 : 4 : 13 : 18 : 18, et a un poids moléculaire de 53.050.

8. Procédé pour la production des vaccins bivalents selon les revendications 1 et 3 à 7, caractérisé en ce que le ou les antigène(s) purifié(s) obtenu(s) à partir des cultures de Pseudomonas aeruginosa est ou sont filtré(s) stérilement, melangé(s), le cas échéant, avec un adjuvant, tel que $Al(OH)_3$, et, si l'on désire, additioné(s) avec un agent préservatif, tel que thiomersalate, et formulé(s) en une préparation galénique.

9. Procédé pour la production des préparations contenant de l'immunoglobuline G et actives contre les infections par la bactérie Pseudomonas aeruginosa selon la revendication 2, caractérisé en ce que du plasma provenant de donateurs humains immunisés avec le ou les antigène(s) flagella(H) est traité par des précipitants, tel que du sulfate d'ammonium, la fraction précipitée qui contient de l'immunoglobuline G est dissoute, purifiée et formulée en une préparation galénique.

FIG. 1

FIG. 2